# EUROPEAN PATENT SPECIFICATION

(11) **EP 3 706 871 B1**
(45) Date of publication and mention of the grant of the patent: **01.12.2021**
(21) Application number: 18783012.0
(22) Date of filing: 09.10.2018
(51) Int. Cl.: A61Q 5/00, A61K 8/64, A61K 8/97, A61K 38/17

(54) **A HAIR CARE COMPOSITION COMPRISING SILK FIBROIN**
HAARPFLEGEMITTEL MIT SEIDENFIBROIN
COMPOSITION DE SOINS CAPILLAIRES COMPRENANT DE LA FIBROÏNE DE SOIE

(30) Priority: 10.11.2017 WO PCT/CN2017/110385; 13.12.2017 EP 17206971
(43) Date of publication of application: 16.09.2020
(73) Proprietor: Unilever Global IP Limited, Wirral, CH62 AZD (GB); Unilever IP Holdings B.V., 3013 AL Rotterdam (NL)
(72) Inventor: CAO, Han, Shanghai 200335 (CN); CHEN, Hong, Shanghai 200433 (CN); CHEN, Xin, Shanghai 200433 (CN); PRAMANIK, Amitava, Bangalore 560066 (IN); SHAO, Zhengzhong, Shanghai 200433 (CN); YAO, Jinrong, Shanghai 200433 (CN); ZHOU, Weizheng, Shanghai 200335 (CN)
(74) Representative: Chisem, Janet
(86) International application number: PCT/EP2018/077424
(87) International publication number: WO 2019/091682

(56) References cited:
- WO-A2-2004/073644
- CN-A- 106 420 423
- CN-A- 106 726 922

## Description

### Field of the invention

This invention relates to a hair care composition for reducing hair breakage, and delivering hair repair, lubrication, strengthening and smoothness benefit.

### Background of the invention

The invention relates to a hair care composition, that ensures lesser amount of hair breakage, softer feel and enhancing the repair of broken hair. The present inventors have achieved this through a combination of specific silk fibroins and specific cationic compounds, preferably in an emulsion medium.

Silk fibroins are kind of fibrous proteins present in silk, which is like keratin (which is the main component of hair) in terms of fibrous structure. Besides, silk fibroin has good affinity to keratin due to intermolecular interactions such as hydrogen bonding and hydrophobic interactions. Generally, there are two major types of secondary structures in silk fibroin, e.g. random coil which is water soluble and β-Sheet which is water insoluble. In the present invention, a variety of regenerated silk fibroin proteins or peptides have been developed through controlled degumming, hydrolyzing and dissolving processes. Regenerated silk fibroins were found to deposit well on hair fiber and form robust "layers" due to its good affinity to hair, and eventually deliver good hair damage repair, enhancement and lubricant benefits. However, these materials have been used in hair care products before. Therefore, the present inventors were looking to enhance the efficacy of such compositions. They found that they could do this by combining such silk fibroins with specific cationic compounds, especially by forming stable oil in water emulsions containing oil to show even better hair damage repair and lubrication and smoothness benefits and demonstrate high potential for use in shampoos.

CN106420423 A (Foshan Runxin Washing products Co., 2017) discloses a silicone-free amino acid/fibroin shampoo which comprises the following components in percentage by weight: 8-15% of cocamidopropyl betaine, 4-9% of coconut oil acetal amide, 3-8% of amino acid foaming agent, 3-7% of disodium sulfosuccinate, 0.5-2% of lanolin, 0.3-1.5% of cationic cellulose JR-400, 1-3% of amino acid plant moisturizer, 1-3% of chitosan silk fibroin, 1-4% of polyquaternary amine salt-47, 1-3% of polyquaternary amine salt-22, 0.05-0.1% of methyl isothiazolinone, 0.2-0.4% of citric acid, 0.5-1.5% of essence and the balance of water.

US2015079012 (Tufts University) discloses a silk fibroin-based composition comprising non-hydrolyzed silk fibroin and a humectant agent.

CN101716128 (Guangdong Mingchen Co., 2010) discloses a transparent conditioning shampoo composition, comprising the components in percent by weight: 5.0% to 50.0% of surface active agent, 0.1% to 7.0% of quaternary ammonium salt or amido amine and the balance of carrier with water, wherein the cationic quaternary ammonium salt or the amido amine of special structure is prepared into the shampoo composition for realizing the high unification of transparence and conditioning performance in a system; compared with the performance of the prior art and commercially available famous products, the major indexes of combing performance of dry hair and wet hair, the softness of the dry hair, luster and the like are obviously enhanced.

EP1531853 (Henkel, 2004) discloses cosmetic preparations containing synergistically effective active substance complex made of sericine and fibroin and/or the derivatives thereof for the treatment of skin and hair.

CN106726922 A (Tian E Quansheng Bee Ind Tech Co Ltd, 2017) discloses a kind of shampoo containing silk fibroin amino acid, cetearyl glucoside, coconut oil sodium acetate, rosemary oil, jasmine essential oil, essence, etc. Such silk fibroin amino acid is one single ingredient.

The published art listed above do not teach that the combination of specific silk fibroin along with specific cationic compounds selected from oligo chitosan or an amino acid will give highly desirable hair fiber benefits.

It is thus an object of the present invention to provide for a hair care composition with hair repair, lubrication, strengthening and smoothness benefit using skin fibroin.

### Summary of the invention

According to the first aspect of the invention there is provided a hair care composition comprising:
(i) silk fibroin derived from silkworm *Bombyx mori;*
(ii) cationic compound selected from oligo chitosan or an amino acid; and,
(iii) a cosmetically acceptable vehicle.

According to another aspect of the present invention there is provided a method of preparing the composition of the first aspect comprising preparing an aqueous solution of silk fibroin followed optionally by mixing with an oil; followed by homogenizing the mixture with an aqueous solution of cationic compound.

Yet another aspect of the present invention provides for a method of providing benefit to hair comprising the step of applying a composition of the first aspect to the desired surface, wherein the benefit includes reduced breakage, increased smoothness and lubrication and good hair damage repair.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. In other words, in specifying any ranges of values, any particular upper value can be associated with any particular lower value.

The disclosure of the invention, as found herein, is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

By 'A Hair Care Composition" as used herein, is meant to include a composition for topical application to hair and/or scalp of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, or bar. Non-limiting examples of such compositions include leave-on hair lotions, creams, and wash-off shampoos, conditioners, shower gels, or toilet bar. The composition of the present invention is preferably a wash-off composition, especially preferred being a shampoo or a conditioner.

The hair care composition of the invention comprises silk fibroin derived from silkworm *Bombyx mori;* cationic compound selected from oligo chitosan or an amino acid; and, a cosmetically acceptable vehicle.

Silk fibroin for use in the present invention is derived from silkworm *Bombyx mori.*

There are two types of silk proteins in *Bombyx mori* silkworm cocoon, one is silk fibroin and the other is sericin. Silk fibroin is purified from sericin by boiling silk cocoons in hot water or alkaline solution (called degumming process). Silk fibroin consists of a light (L) chain polypeptide and a heavy (H) chain polypeptide linked together via a disulfide bond. H-chains form discrete β-sheet crystallites serving as the main structural component responsible for the superior mechanical properties of silk fibroin based materials, while L-chain plays little mechanical role as its size is much smaller than H-chain. In terms of amino acid composition, the H-chain comprises primarily of glycine (43%), alanine (30%) and serine (12%). Upon degumming the raw silk to remove sericin, the obtained fibroin fibers appear shiny and feel soft, and are highly sought after in the textile industry. Moreover, the fibroin fibers are endowed with a combination of attractive strength, toughness, biocompatibility, biodegradability and thermal stability. With its versatile process ability, various silk fibroin formats can be regenerated from dissolved fibroin fibers (i.e., fibroin solution). These formats/matrices serve most notably as surgical sutures, carriers for controlled release and drug delivery, also scaffolds for tissue engineering.

The silk fibroin for use in the composition of the present invention preferably has a molecular weight of at least 30 kDa, most preferably at least 60 kDa. The molecular weight is preferably in the range of 30 to 350 kDa, preferably in the range of 60 to 250 kDa. The silk fibroin meeting the molecular weight of 30 to 350 kDa is prepared by first degumming i.e. removing sericin from silkworm cocoon to get fibroin fiber. This is followed by a dissolving step where fibroin fiber is dissolved in aqueous solution. The degumming of silk cocoon is preferably carried out using sodium carbonate or sodium bicarbonate solution, and the dissolving step is carried out using lithium bromide aqueous solution or calcium chloride/ethanol/water ternary solution. The next step usually is to dialyze above solution against deionized water to remove inorganic ions. The silk fibroin meeting the molecular weight of 0.2 to 30 kDa is prepared by hydrolysis or enzymolysis of above silk fibroin with molecular weight of 30 to 350 kDa, preferably prepared by enzymolysis wiith α-chymotrypsin or elastase.

Silk fibroin is preferably included in 0.1 to 10 wt%, more preferably 0.1 to 5 wt%, most preferably 0.1 to 2 wt% by weight of the composition.

The composition of the invention comprises a cationic compound selected from oligo chitosan or an amino acid. Chitosans generally have the structure as given below:

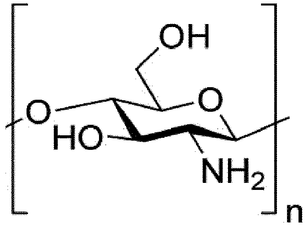

Chitosan is a biopolymer produced from chitin, which is one of the most abundant biopolymers in nature after cellulose. Chitosan has many chemical or physical qualities such as high moisture-absorptive and moisture-retaining properties, film-forming, emulsifying, thickening, and antibacterial activity. It has been widely used in many industrial and biomedical applications including waste water treatment, chromatographic support, enzyme immobilization and carriers for controlled drug delivery. Oligo chitosan is a kind of oligosaccharide made from chitosan by chemical or enzymatic or microwave decomposing methods. Generally, oligo chitosan has 2 to 20 repeating chitin units, with the molecular weight less than 3200 Da. Compared with chitosan, oligo chitosan oligosaccharide has many advantages like (i) better water solubility and moderate water absorption properties; (ii) lower viscosity of the high concentration aqueous solution, which is beneficial for formulation (iii) better stability and safety; and (iv) wider spectrum of bioactivity, such as tumor growth inhibition, enhancing immunity, antibacterial and antifungal activity.

The composition of the invention preferably has oligo chitosan of 2 to 20 repeating chitin units, more preferably 3 to 8 chitin units.

Alternatively, the cationic compound may be an amino acid. Suitable amino acids include arginine, histidine, lysine, cysteine, glutamine, glutamic acid, phenylalanine, proline, tryptophan, tyrosine, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Preferably the amino acid as per the invention is selected from lysine, arginine, histidine, glutamine, phenylalanine, proline, serine, tryptophan and tyrosine and mixtures thereof. The most suitable amino acids for the use according to the invention are lysine or arginine. The composition preferably comprises 0.05 to 5 wt%, preferably 0.1 to 2 wt% of the cationic compound.

The composition of the invention comprises a cosmetically acceptable vehicle. The most preferred vehicle is an emulsion. Emulsion are mixtures, usually thermodynamically stable mixtures of oil and water. The emulsion may be an oil in water emulsion or a water in oil emulsion or a more complex emulsion. Emulsions are generally stabilized using emulsifiers of which organic surfactants are the most common type. The emulsion as per the present invention is preferably an oil in water emulsion. It is interesting that as per the present invention the silk fibroin acts as the emulsifier to stabilize the emulsion when so prepared. The oil used to prepare the emulsion is preferably selected from vegetable oil, mineral oil, silicone oil or mixtures thereof. The vegetable oil is preferably selected from one or more of palm oil, canola oil, corn oil, neem oil, olive oil, cottonseed oil, coconut oil, fractionated coconut oil, nut oils, safflower oil, sesame oil, soybean oil or sunflower oil. Oil is preferably included in 0.1 to 20 wt% preferably 0.5 to 15 wt% by weight of the composition.

As per an especially preferred aspect of the invention, the composition is a shampoo, a hair conditioner or a leave on product.

The composition of the invention especially when formulated as a shampoo preferably comprises an anionic surfactant e.g. an alkyl sulphate and/or ethoxylated alkyl sulfate surfactant. These anionic surfactants are preferably present at a level of from 2 to 16%, more preferably from 3 to 16% by weight of the composition. Preferred alkyl sulfates are C8 to 18 alky sulfates, more preferably C12 to 18 alkyl sulfates, preferably in the form of a salt with a solubilizing cation such as sodium, potassium, ammonium or substituted ammonium. Examples are sodium lauryl sulfate (SLS) or sodium dodecyl sulfate (SDS).

Preferred alkyl ether sulfates are those having the formula: RO (CH2CH2O) nSO₃M; wherein R is an alkyl or alkenyl having from 8 to 18 (preferably 12 to 18) carbon atoms; n is a number having an average value of greater than at least 0.5, preferably between 1 and 3, more preferably between 2 and 3; and M is a solubilizing cation such as sodium, potassium, ammonium or substituted ammonium. An example is sodium lauryl ether sulfate (SLES).

Preferred ethoxylated alkyl sulfate anionic surfactant is sodium lauryl ether sulfate (SLES) having an average degree of ethoxylation of from 0.5 to 3, preferably 1 to 3.

The composition as per the invention optionally and preferably additionally comprises a betaine surfactant. In a preferred embodiment, the composition comprises from 0.1 to 10 wt%, preferably from 0.5 to 8 wt%, more preferably from 1 to 5 wt% of a betaine surfactant, preferably an alkyl amidopropyl betaine, for example cocamidopropyl betaine.

Shampoo compositions according to the invention may comprise one or more further anionic cleansing surfactants which are cosmetically acceptable and suitable for topical application to the hair.

Examples of further suitable anionic cleansing surfactants are the alkaryl sulphonates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in shampoo compositions of the invention include sodium oleyl succinate, ammonium lauryl sulphosuccinate, sodium lauryl ether sulphosuccinate, sodium dodecylbenzene sulphonate, triethanolamine dodecylbenzene sulphonate, lauryl ether carboxylic acid and sodium N-lauryl sarcosinate.

Suitable preferred additional anionic cleansing surfactants are sodium lauryl ether sulphosuccinate(n)EO, (where n is from 1 to 3), lauryl ether carboxylic acid (n) EO (where n is from 10 to 20).

Mixtures of any of the foregoing anionic cleansing surfactants may also be suitable.

If added, the total amount of anionic cleansing surfactant in shampoo compositions of the invention may generally range from 0.5 to 45 wt%, preferably from 1.5 to 35 wt%, more preferably from 5 to 20 wt%, calculated by total weight anionic cleansing surfactant based on the total weight of the composition.

The hair conditioning composition comprises conditioning surfactants selected from cationic surfactants, used singly or in admixture. Preferably, the cationic surfactants have the formula N⁺R1R2R3R4 wherein R1, R2, R3 and R4 are independently (C1 to C30) alkyl or benzyl. Preferably, one, two or three of R1, R2, R3 and R4 are independently (C4 to C30) alkyl and the other R1, R2, R3 and R4 group or groups are (C1-C6) alkyl or benzyl. More preferably, one or two of R1, R2, R3 and R4 are independently (C6 to C30) alkyl and the other R1, R2, R3 and R4 groups are (C1-C6) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (e.g., oleyl). Alkyl groups are optionally ethoxylated on the alkyl chain with one or more ethyleneoxy groups.

Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant. Yet another preferred cationic surfactant is stearamidopropyl dimethylamine.

The most preferred cationic surfactants for use in the composition are stearamidopropyl dimethylamine, behentrimonium chloride, or stearyl trimethyl ammonium chloride. In conditioners of the invention, the level of cationic surfactant will generally range from 0.1 to 5%, preferably 0.5 to 2.5% by weight of the composition.

Hair conditioning compositions of the invention preferably may also additionally comprise a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups.

Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention will generally range from 0.5 to 10 wt%, preferably from 0.1 to 8 wt%, more preferably from 0.2 to 7 wt%, most preferably from 0.3 to 6 wt% by weight of the composition. The weight ratio of cationic surfactant to fatty alcohol is suitably from 1:1 to 1:10, more preferably from 1:1.5 to 1:8, optimally from 1:2 to 1:5.

According to another aspect of the present invention, the composition may be delivered as a leave on product. In such cases, the additional surfactants and other actives used to deliver the product as a shampoo or a conditioner may not be required. The cosmetically acceptable vehicle in such a product may simply be water or water structured with polymers.

Another aspect of the present invention relates to a method of preparing the composition of the invention comprising preparing an aqueous solution of silk fibroin followed by optionally mixing with an oil; followed by homogenizing the mixture with an aqueous solution of cationic compound.

The composition of the invention, in another aspect provides for a method of providing benefit to hair comprising the step of applying the composition on to the desired surface, wherein the benefit includes reduced breakage, increased smoothness and lubrication and good hair damage repair. The method is preferably non-therapeutic.

The invention will now be illustrated with the help of the following non-limiting examples.

### Examples

### Examples A, B, 1 to 3:

The following emulsions as shown in Table -1 were prepared.

**Table - 1**

| Example | A | B | 1 | 2 | 3 |
|---|---|---|---|---|---|
| Silk Fibroin^{X} (wt%) | 1.0 | 1.0 | 1.0 | 1,0 | 1.0 |
| Palm oil, wt% | 10 | 10 | 10 | 10 | 10 |
| Cationic compound | - | - | Oligochitosan^{Y} | Lysine | Histidine |
| Cationic compound, wt% | 0.2 | 0.2 | 0.2 | 0.2 | 0.2 |
| Water | To 100 | To 100 | To 100 | To 100 | To 100 |
| Hair treatment | Virgin | Bleached | Bleached hair was treated with composition | Bleached hair was treated with composition | Bleached hair was treated with composition |

X - Silk fibroin used here had a molecular weight of 95 kDa. It was prepared using the following procedure:
   Silk Fibroin (SF) solution with high MW (95 ± 1 kDa) was prepared using the following sequence of methods:
   (1) Degumming: boiling raw *Bombyx mori* silkworm cocoons in 0.5 wt % sodium carbonate solution twice (each 20 min) to remove sericin.
   (2) Dissolving: the degummed dry silk was dissolved in 9.5 mol/L lithium bromide aqueous solution at 40 °C for 40 min. (3) Purifying: after being filtered, the solution was dialyzed against deionized water for 72 h at room temperature with a 12-14 kDa molecular weight cutoff dialysis membrane to remove the salt. The dialyzed solution was then clarified by centrifuging at 8,000 rpm for 8 min. The supernatant which was an aqueous SF solution was collected and stored at 4 °C before use.
Y - The oligochitosan used had repeating chitin units in the range of 3 to 8.

The SF emulsion was prepared by mixing SF solution (MW 95 kDa) with palm oil and homogenizing for 3 minutes. The cationic agents were then added into the obtained SF emulsion and homogenized for 1 minute.

### Effect of the composition of the invention on dry friction of hair, number of broken hair, and denaturation temperature

### Dry friction of hair

The dry friction of hair treated with the compositions of the invention was measured as follows.

Bleached hair switches (5 g, 10") were treated with 5 ml of SF emulsions (from Table 1 above) for 1 min (by gently massaging the hair), and the hair was rinsed for 30 seconds, and dried overnight at 20°C / 50% relative humidity.

The data on dry friction of the hair so treated was measured using Texture Analyzer and is given in Table 2 below.

### Number of broken hair:

The number of hair fibers broken after 5000 combing strokes was measured. The data is summarized in Table 2 below.

### Denaturation temperature:

The denaturation temperature of the treated hair fibers was measured using Differential Scanning Calorimetry and the data is summarized for this too in Table 2 below.

**Table 2**

| Example | A | B | 1 | 2 | 3 |
|---|---|---|---|---|---|
| Mean dry friction (g.mm) | 48838 | 62383 | 21287 | 43515 | 43105 |
| Broken hair | 121 | 220 | 12 | 23 | 34 |
| Denaturation temperature, (°C) | 150.2 | 147.1 | 151.5 | 151.8 | 152. 2 |

The data in Table 2 above indicates that the compositions as per the invention (Examples 1 to 3) provide for vastly reduced dry friction indicating very good manageability of the hair as compared to virgin hair and especially compared to bleached hair. Similar behavior is seen for number of broken hair where compositions of the invention are seen to deliver this benefit vastly better. The denaturation temperature which is also an indication of the inherent strength of the hair is seen to be superior (even compared to virgin hair) for samples treated with the compositions of the invention.

## Claims

1. A hair care composition comprising:
(i) silk fibroin derived from silkworm *Bombyx mori*;
(ii) cationic compound selected from oligo chitosan or an amino acid; and,
(iii) a cosmetically acceptable vehicle;
wherein said silk fibroin has a molecular weight of at least 30 kDa; wherein said oligo chitosan has 2 to 20 repeating chitin units.

2. A composition as claimed in claim 1 wherein the silk fibroin has a molecular weight of at least 60 kDa.

3. A composition as claimed in claim 1 or 2 wherein said oligo chitosan has 3 to 8 chitin units.

4. A composition as claimed in any one of the preceding claims 1 to 3 wherein the amino acid is selected from lysine or arginine.

5. A composition as claimed in any one of the preceding claims 1 to 4 wherein the cosmetically acceptable vehicle is an emulsion.

6. A composition as claimed in claim 5 wherein the oil in the emulsion is selected from vegetable oil, mineral oil, silicone oil or mixtures thereof.

7. A composition as claimed in claim 6 wherein the vegetable oil is selected from one or more of palm oil, canola oil, corn oil, neem oil, olive oil, cottonseed oil, coconut oil, fractionated coconut oil, nut oils, safflower oil, sesame oil, soybean oil or sunflower oil.

8. A composition as claimed in claim 6 or 7 comprising 0.1 to 20 wt%, preferably 0.5 to 15 wt% of oil.

9. A composition as claimed in any one of the preceding claims1 to 8 comprising 0.1 to 10 wt%, preferably 0.1 to 5 wt%, most preferably 0.1 to 2 wt% of silk fibroin by weight of the composition.

10. A composition as claimed in any one of the preceding claims 1 to 9 comprising 0.05 to 5 wt% of the cationic compound.

11. A composition as claimed in any one of the preceding claims 1 to 10 wherein pH of said composition is 3 to 8.

12. A composition as claimed in any one of the preceding claims 1 to 10 wherein said composition is a shampoo, hair conditioner or a leave on product.

13. A method of preparing the composition as claimed in any one of the preceding claims 1 to 12 comprising preparing an aqueous solution of silk fibroin followed optionally by mixing with an oil; followed by homogenizing the mixture with an aqueous solution of cationic compound.

14. A non-therapeutic method of providing benefit to hair comprising a step of applying a composition as claimed in any one of the preceding claims1 to 12 on to the desired surface, wherein the benefit includes reduced breakage, increased smoothness and lubrication and good hair damage repair.

## Patentansprüche

1. Haarpflegezusammensetzung, umfassend:
(i) Seidenfibroin, das von der Seidenraupe Bombyx mori abgeleitet ist;
(ii) kationische Verbindung, ausgewählt aus Oligochitosan oder einer Aminosäure; und
(iii) ein kosmetisch verträgliches Vehikel;
wobei das Seidenfibroin ein Molekulargewicht von mindestens 30 kDa aufweist; wobei das Oligochitosan 2 bis 20 Chitin-Wiederholungseinheiten aufweist.

2. Zusammensetzung wie in Anspruch 1 beansprucht, wobei das Seidenfibroin ein Molekulargewicht von mindestens 60 kDa aufweist.

3. Zusammensetzung wie in Anspruch 1 oder 2 beansprucht, wobei das Oligochitosan 3 bis 8 Chitineinheiten aufweist.

4. Zusammensetzung wie in irgendeinem der vorhergehenden Ansprüche 1 bis 3 beansprucht, wobei die Aminosäure aus Lysin oder Arginin ausgewählt ist.

5. Zusammensetzung wie in irgendeinem der vorhergehenden Ansprüche 1 bis 4 beansprucht, wobei das kosmetisch verträgliche Vehikel eine Emulsion ist.

6. Zusammensetzung wie in Anspruch 5 beansprucht, wobei das Öl in der Emulsion ausgewählt ist aus Pflanzenöl, Mineralöl, Siliconöl oder Mischungen davon.

7. Zusammensetzung wie in Anspruch 6 beansprucht, wobei das Pflanzenöl ausgewählt ist aus einem oder mehreren von Palmöl, Rapsöl, Maisöl, Neemöl, Olivenöl, Baumwollsamenöl, Kokosnussöl, fraktioniertem Kokosnussöl, Nussölen, Safloröl, Sesamöl, Sojaöl oder Sonnenblumenöl.

8. Zusammensetzung wie in Anspruch 6 oder 7 beansprucht, umfassend 0,1 bis 20 Gew.-%, bevorzugt 0,5 bis 15 Gew.-%, Öl.

9. Zusammensetzung wie in irgendeinem der vorhergehenden Ansprüche 1 bis 8 beansprucht, umfassend 0,1 bis 10 Gew.-%, bevorzugt 0,1 bis 5 Gew.-%, am meisten bevorzugt 0,1 bis 2 Gew.-%, Seidenfibroin, bezogen auf das Gewicht der Zusammensetzung.

10. Zusammensetzung wie in irgendeinem der vorhergehenden Ansprüche 1 bis 9 beansprucht, umfassend 0,05 bis 5 Gew.-% der kationischen Verbindung.

11. Zusammensetzung wie in irgendeinem der vorhergehenden Ansprüche 1 bis 10 beansprucht, wobei der pH-Wert der Zusammensetzung 3 bis 8 beträgt.

12. Zusammensetzung wie in irgendeinem der vorhergehenden Ansprüche 1 bis 10 beansprucht, wobei die Zusammensetzung ein Shampoo, ein Haarconditioner oder ein Leave-on-Produkt ist.

13. Verfahren zur Herstellung der Zusammensetzung wie in irgendeinem der vorhergehenden Ansprüche 1 bis 12 beansprucht, umfassend das Herstellen einer wässrigen Lösung von Seidenfibroin, gefolgt von gegebenenfalls Mischen mit einem Öl; gefolgt von Homogenisieren der Mischung mit einer wässrigen Lösung von kationischer Verbindung.

14. Nichttherapeutisches Verfahren zur Bereitstellung von Nutzen für das Haar, umfassend einen Schritt des Aufbringens von einer Zusammensetzung wie in irgendeinem der vorhergehenden Ansprüche 1 bis 12 beansprucht auf die gewünschte Oberfläche, wobei der Nutzen einen verringerten Bruch, eine erhöhte Glätte und Schmierung und eine gute Reparatur von Haarschäden beinhaltet.

## Revendications

1. Composition pour le soin des cheveux comprenant :
(i) de la fibroïne de soie dérivée de *Bombyx mori* de ver à soie ;
(ii) un composé cationique choisi parmi un oligo chitosan ou un acide aminé ; et,
(iii) un véhicule cosmétiquement acceptable ;
dans laquelle ladite fibroïne de soie présente une masse moléculaire d'au moins 30 kDa ; dans laquelle ledit oligo chitosan présente de 2 à 20 unités chitine répétitives.

2. Composition selon la revendication 1, dans laquelle la fibroïne de soie présente une masse moléculaire d'au moins 60 kDa.

3. Composition selon la revendication 1 ou 2, dans laquelle ledit oligo chitosan présente de 3 à 8 unités chitine.

4. Composition selon l'une quelconque des revendications 1 à 3 précédentes, dans laquelle l'acide aminé est choisi parmi la lysine ou l'arginine.

5. Composition selon l'une quelconque des revendications 1 à 4 précédentes, dans laquelle le véhicule cosmétiquement acceptable est une émulsion.

6. Composition selon la revendication 5, dans laquelle l'huile dans l'émulsion est choisie parmi de l'huile végétale, huile minérale, huile de silicone ou des mélanges de celles-ci.

7. Composition selon la revendication 6, dans laquelle l'huile végétale est choisie parmi une ou plusieurs parmi l'huile de palme, huile de canola, huile de maïs, huile de margousier, huile d'olive, huile de coton, huile de noix de coco, huile de noix de coco fractionnée, huiles de noix, huile de carthame, huile de sésame, huile de soja ou huile de tournesol.

8. Composition selon la revendication 6 ou 7 comprenant de 0,1 à 20 % en masse, de préférence de 0,5 à 15 % en masse d'huile.

9. Composition selon l'une quelconque des revendications 1 à 8 précédentes comprenant de 0,1 à 10 % en masse, de préférence de 0,1 à 5 % en masse, encore mieux de 0,1 à 2 % en masse de fibroïne de soie en masse de la composition.

10. Composition selon l'une quelconque des revendications 1 à 9 précédentes comprenant de 0,05 à 5 % en masse du composé cationique.

11. Composition selon l'une quelconque des revendications 1 à 10 précédentes, dans laquelle le pH de ladite composition est de 3 à 8.

12. Composition selon l'une quelconque des revendications 1 à 10 précédentes, dans laquelle ladite composition est un shampoing, un après-shampoing ou un produit sans rinçage.

13. Procédé de préparation de la composition selon l'une quelconque des revendications 1 à 12 précédentes comprenant la préparation d'une solution aqueuse de fibroïne de soie suivie éventuellement par le mélange avec une huile ; suivie par une homogénéisation du mélange avec une solution aqueuse de composé cationique.

14. Procédé non-thérapeutique fournissant un bénéfice aux cheveux comprenant une étape d'application d'une composition selon l'une quelconque des revendications 1 à 12 précédentes sur la surface souhaitée, dans lequel le bénéfice inclut des cassure réduite, lissé et lubrification augmentés et bonne réparation des détériorations des cheveux.
